# EUROPÄISCHE PATENTSCHRIFT

(11) **EP 2 883 448 B1**
(45) Veröffentlichungstag und Bekanntmachung des Hinweises auf die Patenterteilung: **21.02.2018**
(21) Anmeldenummer: 14195654.0
(22) Anmeldetag: 01.12.2014
(51) Int. Cl.: A01K 39/012

(54) **Kontrollfutterschale und Fütterungsanordnung für die Geflügelhaltung**
Control feed tray and feed assembly for poultry
Mangeoire commandée et système d'alimentation pour l'élevage de volaille

(30) Priorität: 12.12.2013 DE 202013010980 U
(43) Veröffentlichungstag der Anmeldung: 17.06.2015
(73) Patentinhaber: Big Dutchman International GmbH, 49377 Vechta (DE)
(72) Erfinder: Otto-Luebker, Friedrich, 49635 Badbergen Ortsteil Vehs (DE)
(74) Vertreter: Eisenführ Speiser

(56) Entgegenhaltungen:
- US-A- 3 598 087
- US-A- 5 517 944
- US-A1- 2009 078 209
- US-A1- 2013 305 994

## Beschreibung

Die Erfindung betrifft eine Kontrollfutterschale für den Einsatz in einer Fütterungsanordnung für die Geflügelhaltung, insbesondere in Ställen, sowie eine Fütterungsanordnung für die Geflügelhaltung mit einer solchen Kontrollfutterschale.

In der modernen, insbesondere industriellen, Geflügelhaltung, werden Geflügeltiere meist in großer Anzahl in Ställen gehalten. Zur Fütterung der Geflügeltiere werden Fütterunganordnungen eingesetzt, bei denen über Förderleitungen Futter zu einer Vielzahl von Futterschalen gefördert wird. Zur Steuerung der Futterzufuhr zu den Futterschalen über die Förderleitungen werden sogenannte Konkrollfutterschalen eingesetzt, die mit einem Füllstandssensor ausgestattet sind, der in Abhangigkeit vom Füllstand in der Kontrollfutterschale ein Antriebssteuerungssignal generiert, über das die Zufuhr von Futter zu den Futterschalen gesteuert werden kann.

In existierenden Fütterungsanordnungen mit bekannten Kontrollfutterschalen kommt es in der Praxis jedoch häufig vor, dass einzelne oder eine Vielzahl der Futterschalen in einem Stall nicht oder nicht ausreichend mit Futter gefüllt sind, was nachteilig für den Masterfolg ist.

Aus US 2013/0305994 A1 ist eine Futterschale bekannt, bei der die Futtermenge und die Zudosierungsfrequenz basiert auf messbaren Parametern und der erwarteten Wachstumskurve des Tierbestands steuerbar sind.
Aus US 209 310036976 A1 ist eine Futterschale vorbekannt, welche einen Füllstandssensor Im Fallrohr aufweist, der ein Füllstandsignal für einen Leerzustand und ein Füllstandsignal für einen gefüllten Zustand in dem Fallrohr erzeugt.
Aus US 2009/0078209 A1 ist ein Fütterungssystem bekannt, bei dem aus einem Lichtsystem Licht in eine Fütterungsvorrichtung abgegeben wird, um die Futteraufnahme der Tiere zu erhöhen.

Aus US 5,517,944 ist eine Fütterungseinrichtung bekannt, die Schallemissionsmittel aufweist, um während einer Fütterungsperiode Geräusche als Anreize abzugeben.
Aus US 3,598,087 ist eine Fütterungsanordnung mit mehren Futterschalen bekannt, bei der die Zudosierung der Futterschalen anhand mindestens eines Füllstandsensors gesteuert wird.

Es ist daher eine Aufgabe der vorliegenden Erfindung, eine Kontrollfutterschale und eine Fütterungsanordnung bereitzustellen, die einen oder mehrere der genannten Nachteile vermindern oder beseitigen.

Diese Aufgabe wird gelöst durch eine Kontrollfutterschale für den Einsatz in einer Fütterungsanordnung für die Geflügelhaltung, umfassend ein Montageelement zur Montage der Futterschale an einer Förderleitung; einen Futterteller; einen Futterschacht, über den Futter aus einer Förderleitung in den Futterteller gelangen kann; einen Füllstandssensor, der angeordnet und ausgebildet ist, ein Antriebsteuersignal zu generieren; gekennzeichnet durch eine Signaleinheit, die angeordnet und ausgebildet ist, ein von Geflügeltieren wahrnehmbares Futtersignal abzugeben, sodass die Kontrollfutterschale stärker frequentiert wird als die Futterschale. Diese Kontrollfutterschale ist Teil einer Fütterungsanordnung, gemäß Anspruch 1, die neben der Kontrollfutterschale weitere Futterschalen aufweist. Von den übrigen, normalen Futterschalen unterscheidet sich eine Kontrollfutterschale insbesondere durch den Füllstandssensor.

Die Kontrollfutterschale ist vorzugsweise wie folgt aufgebaut: Sie weist ein Montageelement auf, mit dem die Kontrollfutterschale freischwingend oder fest an einer, meist horizontal im Stall verlaufenden, Förderleitung befestigt werden kann. Von der Förderleitung gelangt Futter, vorzugsweise über Öffnungen in der Förderleitung, über einen Futterschacht in einen Futterteller, aus dem die Geflügeltiere Futter aufnehmen können.

Der Füllstandssensor kann, vorzugsweise in Abhängigkeit vom Füllstand des Futters in der Kontrollfutterschale, insbesondere im Futterschacht der Kontrollfutterschale, ein Antriebssteuersignal erzeugen und vorzugsweise direkt oder indirekt, kabelgebunden oder kabellos an einen Förderantrieb einer Förderleitung übermitteln. Über das Antriebssteuersignal kann der Förderantrieb angesteuert werden, insbesondere aktiviert und/oder gestoppt werden.

Das Antriebssteuersignal wird vorzugsweise in Abhängigkeit vom Füllstand in der Kontrollfutterschale, d. h. in Abhängigkeit von der Menge des in der Kontrollfutterschale zur Verfügung stehenden Futters, ermittelt. Beispielsweise kann der Füllstandssensor ein Antriebssteuersignal generieren, wenn ein bestimmter Mindestfüllstand und/oder ein bestimmter Maximalfüllstand unter- und/oder überschritten wird. Es können auch zwei Füllstandssensoren oder ein Füllstandssensor mit zwei Messfühler vorgesehen sein, die unterschiedliche Füllstände und deren Über- bzw. Unterschreitung detektieren.

In einer Fütterungsanordnung existiert in der Regel mindestens eine Förderleitung, die beispielsweise als Förderrohr, Förderspirale oder Förderschnecke ausgebildet sein kann, die von einem Förderantrieb angetrieben wird, so dass Futter vorzugsweise von einem Futterbehälter, ggf. über eine Futtermaschine, durch die Förderleitung transportiert und über Öffnungen in der Förderleitung an die Futter- und Kontrollfutterschalen abgegeben werden kann. Eine Kombination aus Futterbehälter, Förderleitung mit Förderantrieb und mindestens einer Futterschale sowie mindestens einer Kontrollfutterschale kann auch als Futterlinie bezeichnet werden. In einem Stall für die Geflügelhaltung gibt es in der Regel mehrere, vorzugsweise unabhängig voneinander gesteuerte bzw. geregelte Futterlinien, die sich vorzugsweise im Wesentlichen über die gesamte Stalllänge erstrecken. Die Futterlinien sind in der Regel entlang der Längsrichtung des Stalls orientiert. Neben individuellen, vorzugsweise parallel angeordneten Futterlinien sind auch Kreisläufe als Fördersystem einsetzbar.

Am Stallanfang können die Futterlinien durch eine Verteilleitung mit einem Verteilantrieb verbunden sein, über die von einem Futtersilo aus Futter zu den einzelnen Förderleitungen der Futterlinien gefördert werden kann. Es können auch mehrere Verteilleitungen mit mehreren Verteilantrieben vorgesehen sein.

Das Antriebssteuersignal einer Kontrollfutterschale kann vorzugsweise dazu dienen, einen Förderantrieb einer Förderleitung, mehrere Förderantriebe mehrerer Förderleitungen, einen Verteilantrieb einer Verteilleitung und/oder mehrere Verteilantriebe mehrerer Verteilleitungen anzusteuern. Ein Verteilantrieb einer Verteilleitung kann beispielsweise auch über Füllstandssensoren in den jeweiligen Futterbehältern gesteuert werden.

In den Futterlinien wird das Futter in der Regel beginnend vom Stallanfang an zum Stallende hin durch die Förderleitung von Futterschale zu Futterschale gefördert. Die Förderung kann so ausgestaltet sein, dass, sobald eine Futterschale komplett gefüllt ist, zur nächsten Futterschale einer Futterlinie überfördert wird. Als Kontrollfutterschale ist vorzugsweise eine Futterschale am Ende einer Futterlinie ausgebildet, beispielsweise die letzte Futterschale, d. h. die am Stallende einer Förderleitung angeordnete Futterschale. Es können aber auch andere Futterschalen als Kontrollfutterschalen ausgebildet sein.

Der Füllstandssensor überwacht den Füllstand des Futters in der Kontrollfutterschale, vorzugsweise kontinuierlich, und generiert ein Antriebssteuerungssignal, über das der Förderantrieb einer Futterlinie und/oder der Verteilantrieb der Verteilleitung aktiviert oder gestoppt werden kann.

Die Erfindung beruht unter anderem auf der Erkenntnis, dass die Geflügeltiere einzelne Futterschalen gegenüber anderen bevorzugen und verschiedene Futterschalen unterschiedlich oft besucht werden. Insbesondere dann, wenn die als Kontrollfutterschalen ausgebildeten Futterschalen nicht häufig genug von den Geflügeltiere frequentiert werden, kann es dazu kommen, dass in den Kontrollfutterschalen der Futterstand hoch bleibt und der Füllstandssensor daher einen ausreichenden Füllstand detektiert und kein Antriebssteuerungssignal zur Aktivierung der Förderantriebe generiert. Dies kann zur Folge haben, dass die übrigen Futterschalen, die häufiger frequentiert werden, vor der Kontrollfutterschale von den Geflügeltieren geleert werden und über einen längeren Zeitraum nicht wieder befüllt werden. Damit steht den Geflügeltieren zumindest an diesen Futterschalen kein oder nur sehr wenig Futter zur Verfügung, was sich negativ auf den Masterfolg auswirkt. In Fütterungsanordnungen mit mehreren Futterlinien kann bereits eine nicht oder schlecht frequentierte Kontrollfutterschale Auswirkungen auf die Beschickung mehrerer Futterlinien haben, da diese in der Regel über eine gemeinsame Verteilleitung beschickt werden. So kann beispielsweise eine unterdurchschnittliche Futterabnahme an einer Kontrollfutterschale der letzten Futterlinie eines Stalls dazu führen, dass eine Vielzahl von Futterschalen im gesamten Stall unterversorgt ist.

Erfindungsgemäß ist in der Kontrollfutterschale daher eine Signaleinheit vorgesehen, die ein Futtersignal abgeben kann. Dieses Futtersignal ist von den Geflügeltieren wahrnehmbar und vorzugsweise so gestaltet, dass es auf die Geflügeltiere attraktiv wirkt. Durch dieses Futtersignal können die Geflügeltiere angeregt werden, die Kontrollfutterschale häufiger zu besuchen. Auf diese Weise kann sichergestellt werden, dass die Kontrollfutterschale stärker frequentiert wird. Durch eine höhere Frequentierung der Kontrollfutterschale können die beschriebenen Nachteile der Unterversorgung übriger Futterschalen reduziert oder beseitigt werden.

Das Futtersignal wird von der Signaleinheit vorzugsweise in Richtung der Umgebung, d. h. in Richtung der Geflügeltiere abgegeben. Von einer Längsachse der Kontrollfutterschale aus gesehen, die meist der Vertikalen entspricht, wird das Futtersignal vorzugsweise in radialer Richtung in einem Bereich von 180 bis 360 Grad ausgestrahlt, um möglichst rundum bzw. in einem möglichst großen Kreissegment von den Geflügeltieren wahrgenommen werden zu können. Ferner ist die Signaleinheit vorzugsweise ausgebildet, dass Futtersignal in einem Winkel zur Horizontalen von -80 bis +80 Grad abzugeben, d. h. sowohl schräg nach unten, zum Stallboden hin, als auch schräg nach oben, zur Stalldecke hin. Die obere Grenze kann vorzugsweise auch bei 0 Grad (entsprechend der Horizontalen), +5 Grad, +10 Grad, +15 Grad, +20 Grad, +25 Grad, +30 Grad, +45 Grad, oder +60 Grad liegen. Die untere Grenze kann vorzugsweise bei -70 Grad, -60 Grad, -50 Grad, -45 Grad, -40 Grad, -30 Grad, -25 Grad, -20 Grad, -15 Grad, -10 Grad, -5 Grad liegen. Die Reichweite und Richtung des von der Signaleinheit abzugebenden Futtersignals ist vorzugsweise auf die jeweiligen Geflügeltiere, beispielsweise Küken, Enten, Masthähnchen oder Puten, und insbesondere deren Größe abgestimmt.

Die Signaleinheit ist vorzugsweise ausgebildet, ein optisches, akustisches und/oder olfaktorisches Futtersignal und/oder ein Vibrations-Futtersignal abzugeben. Ferner ist die Signaleinheit vorzugsweise ausgebildet, als Futtersignal eine, vorzugsweise elektromagnetische, Strahlung abzugeben, insbesondere Licht- und/oder Wärmestrahlung abzugeben und/oder ein von Geflügeltieren wahrnehmbares Magnetfeld zu erzeugen. Ferner ist bevorzugt, dass die Signaleinheit eine Strahlungsquelle, insbesondere eine Lichtquelle, und/oder eine Schallquelle, beispielsweise einen Lautsprecher, umfasst. Vorzugsweise ist die Signaleinheit als Kombination einer Lichtquelle mit einem Lautsprecher ausgebildet, um sowohl optische als auch akustische Futtersignale abgeben zu können.

Die Signalquelle kann vorzugsweise Licht im sichtbaren Bereich abgeben, mit einer Wellenlänge von etwa 380 nm bis 780 nm und Frequenzen von etwa 789 THz bis 384 THz. Insbesondere ist bevorzugt, dass von der Signaleinheit als Futtersignal blaues und/oder violettes Licht und/oder ultraviolette Strahlung abgegeben werden kann. Als violettes Licht wird vorzugsweise eine Strahlung mit einer Wellenlänge von 380-420 nm und einer Frequenz von 789,5-714,5 THz abgegeben. Als blaues Licht wird vorzugsweise eine Strahlung mit einer Wellenlänge von 420-490 nm und einer Frequenz von 714,5-612,5 THz abgegeben. Als ultraviolette Strahlung wird vorzugsweise eine Strahlung mit einer Wellenlänge von 100-380 nm und einer Frequenz von 789 THz bis 3 PHz abgegeben. Blaues und/oder violettes Licht und/oder ultraviolette Strahlung sind besonders bevorzugt, da diese besonders attraktiv auf Geflügeltiere wirken können. Ferner kann blaues und/oder violettes Licht und/oder ultraviolette Strahlung den Tieren das Auffinden von Futter bzw. die Futtersuche erleichtern.

Als Lichtquellen können beispielsweise Leuchtdioden (LED) zum Einsatz kommen. Die Signaleinheit, insbesondere die Lichtquelle, kann beispielsweise an einer Oberfläche der Kontrollfutterschale angeordnet sein. Die Signaleinheit, insbesondere die Lichtquelle, kann aber beispielsweise auch (vorzugsweise zusammen mit dem Füllstandssensor) im Inneren eines Futterschachts angeordnet sein, wobei vorzugsweise ein oder mehrere Abschnitte des Futterschachts offen oder lichtdurchlässig bzw. transparent ausgebildet ist bzw. sind. Eine oder mehrere Lichtquellen können beispielsweise auch so angeordnet sein, dass sie in Erstreckungsrichtung einer transparenten Scheibe ausgerichtet sind und an Brechungskanten eine Lichtbrechung hervorrufen.

Eine Signaleinheit, insbesondere eine Lichtquelle kann beispielsweise auch als Leuchtring ausgebildet sein, der vorzugsweise um den Futterschacht gelegt ist. Wenn sich der Futterschacht zur Futterschale hin verbreitert, kann durch die Wahl des Durchmessers des Leuchtrings die Position am Futterschacht festgelegt werden. Die Signaleinheit kann auch mehrere, beispielsweise stabförmig ausgebildete, Signalquellen umfassen, die vorzugsweise ringförmig um den Futterschacht angeordnet sind. Eine oder mehrere Signalquellen können beispielsweise an einer Außenseite des Futterschachts, im Futterschacht, an einem oberen Ende einer Wand des Futterschachts, an Stützarmen der Kontrollfutterschale oder einem Verbindungsring der Stützarme, angeordnet sein. Als Lichtquellen ausgebildete Signaleinheiten stahlen Licht vorzugsweise zumindest teilweise in Richtung eines Futtertellers ab. Falls der Füllstandssensor beispielsweise von außen in den Futterschacht gesteckt ist, kann es auch bevorzugt sein, die Signaleinheit am Füllstandssensor anzubringen.

Zusätzlich oder alternativ kann vorzugsweise auch Infrarotstrahlung als Wärmestrahlung zur Attraktivitätssteigerung eingesetzt werden.

Akustische Signale können beispielsweise (aufgezeichnete oder nachgeahmte) menschliche oder tierische Laute, wie etwa ein Lockruf einer Henne, sein, oder gleichmäßige oder variierende Töne mit gleichbleibender oder variierender Frequenz (beispielsweise Brummtöne). Die Schallerzeugung in der Signalquelle kann beispielsweise mechanisch, aerodynamisch und/oder thermodynamisch erfolgen.

Ferner ist es bevorzugt, dass die Signalquelle ausgebildet ist, ein von Geflügeltieren wahrnehmbares Magnetfeld zu erzeugen. Über ein Magnetfeld, insbesondere ein Magnetfeld, dessen Intensität in etwa dem Erdmagnetfeld entspricht, kann über den Magnetsinn bzw. die Sensitivität bezüglich Magnetfeldern Einfluss auf die Orientierung von Geflügeltieren genommen werden. Dabei ist ein von der Signalquelle erzeugtes Magnetfeld vorzugsweise so ausgebildet, dass Geflügeltiere vermehrt zu der Kontrollschale hingeleitet werden.

Besonders bevorzugt ist ferner, dass die Signaleinheit ausgebildet ist, zwei oder mehrere unterschiedliche Futtersignale abzugeben. Dazu kann die Signaleinheit zwei oder mehrere Signalquellen umfassen, die auch räumlich getrennt angeordnet sein können.

Das Futtersignal und/oder zwei oder mehrere unterschiedliche Futtersignale können von der Signaleinheit vorzugsweise intermittierend und/oder kontinuierlich abgegeben werden. Dazu ist die Signaleinheit vorzugsweise grundsätzlich so ausgebildet, dass Futtersignal bzw. die Futtersignale nur intermittierend, nur kontinuierlich oder sowohl intermittierend als auch kontinuierlich abgeben zu können. In letzterem Fall bedeutet dies, dass die Signaleinheit Futtersignale wahlweise intermittierend oder kontinuierlich abgeben kann.

Ferner ist die Signaleinheit vorzugsweise ausgebildet, ein Futtersignal und/oder zwei oder mehrere unterschiedliche Futtersignale in gleicher und/oder unterschiedlicher Intensität bzw. gleichen oder unterschiedlichen Intensitäten abzugeben. Die Signaleinheit kann ein Futtersignal und/oder zwei oder mehrere unterschiedliche Futtersignale vorzugsweise wahlweise in gleicher oder unterschiedlicher Intensität abgeben. Ferner ist bevorzugt, dass die Signaleinheit ein Futtersignal und/oder zwei oder mehrere unterschiedliche Futtersignale vorzugsweise wahlweise in gleichen oder unterschiedlichen Intensitäten abgeben kann. Beispielsweise kann es bevorzugt sein, dass die Signaleinheit Licht in mehreren Farben aussendet, die verschiedenen Farben aber unterschiedlich intensiv ausgesendet werden. Beispielsweise kann vorzugsweise die Intensität von blauen und/oder violetten Lichtanteilen gegenüber gelben und/oder roten Lichtanteilen erhöht sein.

Besonders bevorzugt ist es, ein optisches Futtersignal mit einem akustischen Futtersignal zu kombinieren, da eine solche Kombination eine besonders gute Attraktivitätssteigerung für die Geflügeltiere darstellen kann. Eine solche Kombination kann beispielsweise darin bestehen, dass ein optisches und ein akustisches Futtersignal gleichzeitig oder abwechselnd abgegeben werden.

In einer weiteren Ausgestaltung ist bevorzugt, dass die Signaleinheit ausgebildet ist, die Dauer und/oder Intensität und/oder Art des Futtersignals und/oder die Dauer einer Pause zwischen Futtersignalabgaben in Abhängigkeit eine Steuerparameters festzulegen und/oder zu variieren. Auf diese Weise kann die Kontrollfutterschale beispielsweise an unterschiedliche Rand- bzw. Einsatzbedingungen angepasst werden.

Variationen in der Futtersignalabgabe, beispielsweise hinsichtlich der Dauer, Unterbrechung, Art und/oder Intensität, können dazu beitragen, dass kein oder nur ein geringer Gewöhnungseffekt auftritt, um so die attraktivitätssteigernde Wirkung des Futtersignals auch über einen längeren Zeitraum, insbesondere über Tage, Wochen, Monate, sicherzustellen.

Ferner ist bevorzugt, dass der Steuerparameter in der Signaleinheit und/oder dem Füllstandssensor abgespeichert ist und/oder von einer externen Datenerfassungs-, Datenverarbeitungs- und/oder Datenspeichereinheit an die Signaleinheit und/oder den Füllstandssensor übermittelt werden kann.

Der Steuerparameter kann also vorzugsweise in der Signaleinheit und/oder dem Füllstandssensor selbst abgespeichert sein und in der Signaleinheit direkt oder über eine kabelgebundene oder kabellose Verbindung mit dem Füllstandssensor abrufbar sein. Es ist auch möglich, den Steuerparameter über eine Benutzereingabe an der Signaleinheit, am Füllstandssensor und/oder an der Kontrollfutterschale einzugeben. Eine weitere Möglichkeit der Bereitstellung des Steuerparameters besteht darin, diesen in einer Datenerfassungs-, Datenverarbeitungs- und/oder Datenspeichereinheit bereitzustellen und von dieser an die Signaleinheit und/oder den Füllstandssensor zu übermitteln, wobei die Übermittlung kabelgebunden oder kabellos erfolgen kann. Es kann beispielsweise auch bevorzugt sein, über externe Datenerfassungs-, Datenverarbeitungs- und/oder Datenspeichereinheiten den Steuerparameter über Benutzervorgaben festzulegen. Ferner ist es jedoch insbesondere bevorzugt, den Steuerparameter aus anderen Datenerfassungs-, Datenverarbeitungs- und/oder Datenspeichereinheiten der Geflügelhaltung bereitzustellen, beispielsweise aus einem zentralen Steuerungscomputer der Geflügelfütterung oder Geflügelhaltung und/oder aus weiteren, insbesondere für die Geflügelhaltung spezifischen, Datenerfassungs-, Datenverarbeitungs- und/oder Datenspeichereinheiten, wie beispielsweise Tierwaagen.

Insbesondere ist es bevorzugt, dass der Steuerparameter einen, zwei oder mehrere Parameter aus der folgenden Gruppe umfasst: Tageszeit, Helligkeit und/oder Temperatur im Stall und/oder der Umgebung, Anzahl der Geflügeltiere, Alter und/oder Gewicht und/oder Größe der Geflügeltiere, Benutzervorgabe, Futter- und/oder Wasserverbrauch, Zeitintervall seit der letzten Ansteuerung eines Förderantriebs und/oder eines Verteilantriebs.

Die Berücksichtigung der Tageszeit als Steuerparameter hat den Vorteil, dass die Frequentierung der Kontrollfutterschale zu bestimmten Fütterungszeiten erhöht werden kann. Die Berücksichtigung der Helligkeit als Steuerparameter hat den Vorteil, dass Licht als Futtersignal insbesondere bei ansonsten wenig Licht im Stall hohe Wirkung zeigt. Ebenso kann eine Wärmestrahlung, beispielsweise Infrarotstrahlung, insbesondere bei niedriger Temperatur im Stall attraktivitätssteigernd wirken. Die Anzahl der Geflügeltiere im Stall bzw. pro Futterlinie kann vorzugsweise ebenfalls als Steuerparameter berücksichtigt werden. Das Gewicht der Geflügeltiere kann vorzugsweise über im Stall befindliche Tierwaagen, wie beispielsweise in der DE 20 2008 007 880 beschrieben, ermittelt werden und von dort, gegebenenfalls über ein zentrales Steuerungssystem, als Steuerparameter bereitgestellt werden. Auch Benutzervorgaben können vorzugsweise als Steuerparameter berücksichtigt werden. Alter und/oder Größe der Geflügeltiere können vorzugsweise ebenfalls aus einem zentralen Steuerungssystem bereitgestellt werden. Als Steuerparameter kann auch der Futter- und/oder Wasserverbrauch, vorzugsweise in einem bestimmten Zeitraum, berücksichtigt werden.

Eine weitere besonders vorteilhafte Gestaltung des Steuerparameters ergibt sich durch die Berücksichtigung der seit der letzten Ansteuerung, insbesondere Aktivierung, eines Förderantriebs und/oder des Verteilantriebs verstrichenen Zeit. Insbesondere ist bevorzugt, das eine maximale Dauer zwischen Futtersignalabgaben nicht überschritten wird. Auch eine solche maximale Dauer zwischen Futtersignalabgaben kann vorzugsweise auch in Abhängigkeit des Steuerparameters festgelegt werden.

Als Steuerparameter kann insbesondere auch eine Kombination der einzelnen Parameter und/oder ein oder mehrere aus diesen Parametern abgeleitete Parameter zum Einsatz kommen und/oder der Steuerparameter über einen Algorithmus unter Berücksichtigung eines oder mehrerer Parameter ermittelt werden.

Insbesondere kann es bevorzugt sein, in einer Futteranordnung, die mehrere Futterlinien mit jeweils mindestens einer Kontrollfutterschale aufweist, die Kontrollfutterschale(n) der verschiedenen Futterlinien über unterschiedliche Algorithmen anzusteuern. Insbesondere die Kontrollfutterschale(n) einer sogenannten Master-Futterlinie, welche die Versorgung auch der übrigen Futterlinien über eine Ansteuerung auch des Verteilantriebs einer Verteilleitung sicherstellt, werden vorzugsweise über einen bestimmten Algorithmus so angesteuert, dass sie häufiger Futtersignale abgeben als Kontrollfutterschale(n) anderer Futterlinien. Dadurch kann verhindert werden, dass durch schlecht besuchter Kontrollfutterschale(n) der Master-Futterlinie eine Unterversorgung der anderen Futterlinien eintritt.

Die Signaleinheit ist ferner vorzugsweise an der Kontrollfutterschale befestigt. Insbesondere bevorzugt ist, dass die Signaleinheit am Füllstandssensor befestigt ist. Die Befestigung der Signaleinheit an der Kontrollfutterschale bzw. am Füllstandssensor ist vorzugsweise lösbar ausgestaltet.

Die Signaleinheit oder zumindest ein Teil davon, beispielsweise eine von mehreren Signalquellen einer Signaleinheit, kann auch als separate Baueinheit ausgebildet und vorzugsweise getrennt von den übrigen Elementen der Kontrollfutterschale angeordnet sein. Als solche separate Baueinheit kann die Signaleinheit beispielsweise getrennt von den übrigen Elementen der Kontrollfutterschale (wie Montageelement, Futterschacht, Futterteller, Füllstandssensor) angeordnet sein und zusammen mit diesen übrigen Elementen die Kontrollfutterschale bilden. Beispielsweise kann die Signaleinheit oder zumindest ein Teil davon neben den übrigen Elementen der Kontrollfutterschale angeordnet sein, etwa an der Förderleitung montiert oder im Bereich der übrigen Elementen der Kontrollfutterschale, beispielsweise daneben oder darunter, auf dem Stallboden angeordnet sein. Die Signaleinheit oder ein Teil davon kann beispielsweise auch an einer Stallwand oder Stalldecke angeordnet sein. Bevorzugt ist, dass die Signaleinheit, wenn sie als separate Baueinheit getrennt von den übrigen Elementen der Kontrollfutterschale angeordnet ist, näher zu den übrigen Elementen der Kontrollfutterschale, vorzugsweise in deren direkter Nähe, angeordnet ist als zu einer anderen, nicht als Kontrollfutterschale ausgebildeten Futterschale. Alternativ kann eine Signaleinheit auch weiter entfernt von den übrigen Elementen der Kontrollfutterschale angeordnet sein. Insbesondere ist es bevorzugt, dass die Signaleinheit, wenn sie als separate Baueinheit getrennt von den übrigen Elementen der Kontrollfutterschale angeordnet ist, derart angeordnet und ausgebildet ist, dass ein von der Signaleinheit abgegebenes Futtersignal so ausgerichtet sind, dass es die Attraktivität der Geflügeltiere auf die Kontrollfutterschale lenkt und/oder insbesondere im Bereich der Kontrollfutterschale, insbesondere im Bereich des Futtertellers, von den Geflügeltieren wahrnehmbar ist. Beispielsweise kann eine Signalquelle ausgebildet sein, ein in Richtung der übrigen Elemente der Kontrollfutterschale gebündeltes Futtersignal abzugeben, wie etwa ein Scheinwerfer oder ein Richtlautsprecher. Beispielsweise kann auch bevorzugt sein, dass die Signaleinheit und der Füllstandssensor als integrierte Baueinheit sind. Die eingangs genannte Aufgabe wird gelöst durch eine Fütterungsanordnung für die Geflügelhaltung, gemäß Anspruch 1, umfassend eine Futterlinie mit einer Förderleitung, einem Förderantrieb, einem Futterbehälter, einer Futterschale und einer zuvor beschriebenen Kontrollfutterschale.

Eine Futterlinie kann vorzugsweise zwei, drei oder mehrerer Futterschalen sowie mindestens eine, möglicherweise jedoch auch zwei, drei oder mehrere Kontrollfutterschalen umfassen. Ferner ist zwischen dem Futterbehälter und der Förderleitung einer Futterlinie vorzugsweise eine Futtermaschine zwischengeschaltet. Eine Fütterungsanordnung kann zwei, drei oder mehrere Futterlinien aufweisen, die vorzugsweise über eine gemeinsame Verteilleitung mit einem Futtersilo verbunden sind, wobei Futter vom Futtersilo vorzugsweise mittels eines Verteilantriebs über die Verteilleitung in die Futterbehälter der Futterlinien gefördert werden kann.

Die erfindungsgemäße Fütterungsanordnung und ihre möglichen Fortbildungen weisen Merkmale auf, die zuvor mit Bezug auf die Kontrollfutterschale beschrieben wurden. Zu den Vorteilen, Ausführungsvarianten und Ausführungsdetails der Fütterungsanordnung und ihrer Fortbildungen wird auf die vorangegangene Beschreibung zu den entsprechenden Merkmalen der Kontrollfutterschale verwiesen.

Gemäß einem weiteren Aspekt wird die eingangs genannte Aufgabe gelöst durch ein Verfahren zur Fütterung von Geflügeltieren in der Geflügelhaltung, gemäß Anspruch 14, umfassend die Schritte: Bereitstellen einer zuvor beschriebenen Fütterungsanordnung; Aktivierung des Förderantriebs zum Fördern von Futter durch die Förderleitung zu der Futterschale und der Kontrollfutterschale, Stoppen des Förderantriebs in Abhängigkeit eines vom Füllstandssensor der Kontrollfutterschale generierten Antriebsteuersignal, Abgeben eines von Geflügeltieren wahrnehmbaren Futtersignals mittels einer Signaleinheit, sodass die Kontrollfutterschale stärker frequentiert wird als die Futterschale. Die Aktivierung des Förderantriebs zum Fördern von Futter durch die Förderleitung zu der Futterschale und der Kontrollfutterschale erfolgt vorzugsweise in Abhängigkeit eines vom Füllstandssensor der Kontrollfutterschale generierten Antriebssteuersignals. Das Verfahren und seine möglichen Fortbildungen weisen Merkmale bzw. Verfahrensschritte auf, die sie insbesondere dafür geeignet machen, für eine Kontrollfutterschale in einer erfindungsgemäßen Fütterungsanordnung und deren entsprechenden Fortbildungen verwendet zu werden. Zu den Vorteilen, Ausführungsvarianten und Ausführungsdetails des Verfahrens und seiner Fortbildungen wird auf die vorangegangene Beschreibung zu den entsprechenden Vorrichtungsmerkmalen verwiesen.

Eine bevorzugte Ausführungsform der Erfindung wird beispielhaft anhand der beiliegenden Figuren beschrieben.

Es zeigen:
- Fig. 1:: einen Geflügelstall mit einer beispielhaften Ausführungsform einer erfindungsgemäßen Fütterungsanordnung;
- Fig. 2:: eine Futterlinie mit einer beispielhaften Ausführungsform einer erfindungsgemäßen Kontrollfutterschale;
- Fig. 3:: eine weitere beispielhafte Ausführungsform einer erfindungsgemäßen Kontrollfutterschale;
- Fig. 4:: einen Teil der in Figur 3 dargestellten Kontrollfutterschale;
- Fig. 5:: eine weitere beispielhafte Ausführungsform einer erfindungsgemäßen Kontrollfutterschale;
- Fig. 6:: eine weitere beispielhafte Ausführungsform einer erfindungsgemäßen Kontrollfutterschale;
- Fig. 7:: eine weitere beispielhafte Ausführungsform einer erfindungsgemäßen Kontrollfutterschale; und
- Fig. 8:: eine weitere beispielhafte Ausführungsform einer erfindungsgemäßen Kontrollfutterschale.

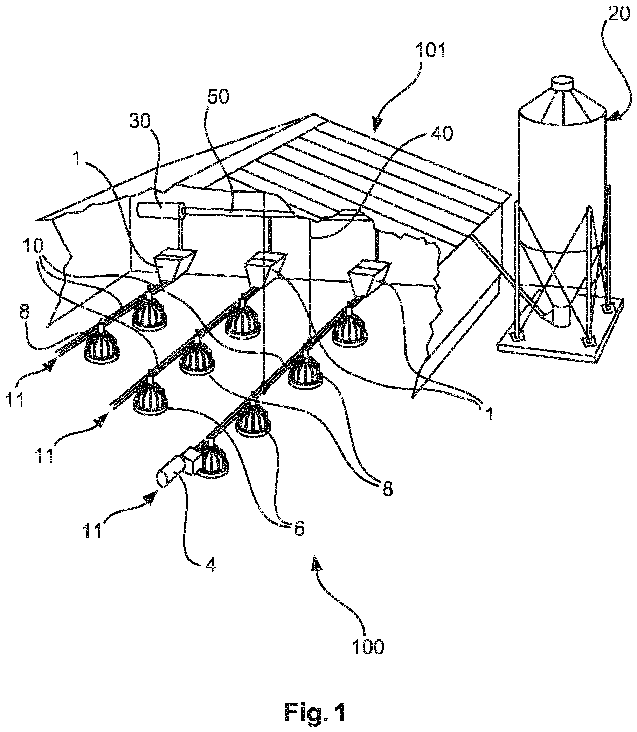

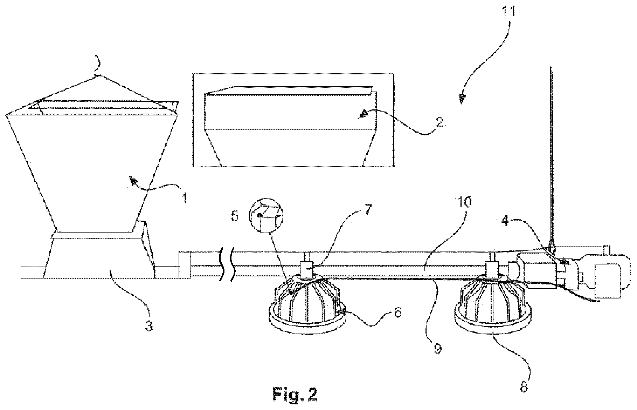

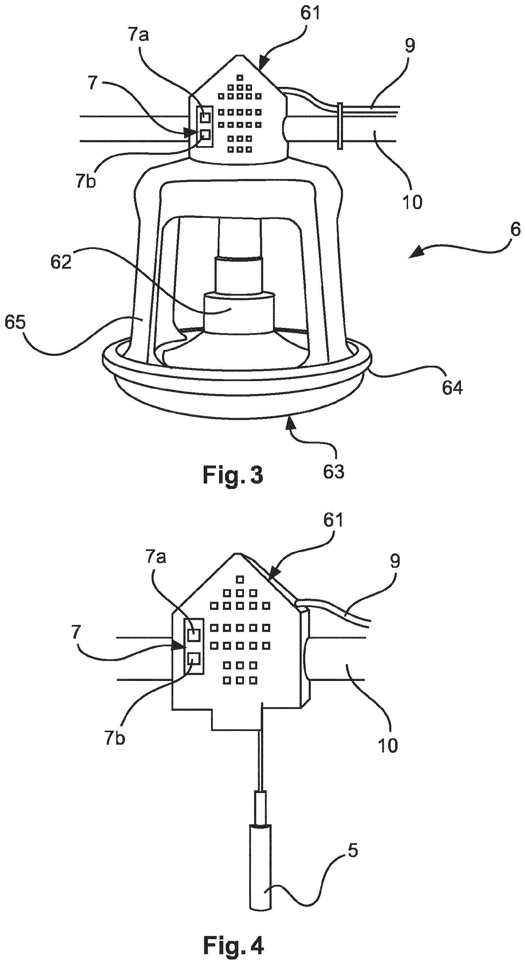

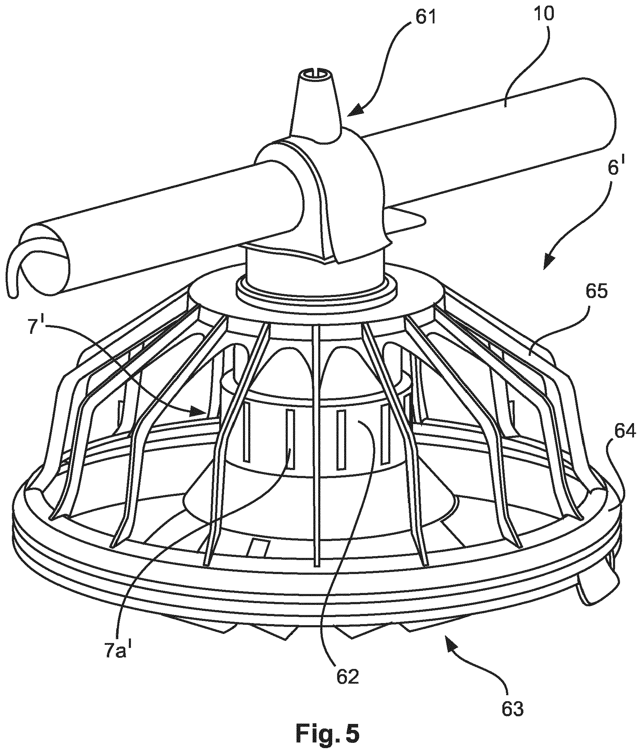

Figur 1 zeigt einen Stall 101 zur Geflügelhaltung mit einer Fütterungsanordnung 100. In Figur 2 ist eine Futterlinie 11 näher dargestellt. In Figur 3 ist ferner eine Ausgestaltung einer Kontrollfutterschale 6 mit in Figur 4 im Detail zu erkennenden Füllstandssensor 5 und einer schematisch dargestellten Signaleinheit 7 gezeigt. Die Figuren 5 bis 8 zeigen weitere beispielhafte Ausführungsformen erfindungsgemäßer Kontrollfutterschalen 6', 6", 6''', 6'''' mit weiteren vorteilhaften Ausgestaltungen von Signaleinheiten 7', 7", 7''', 7''''. Ähnliche oder im wesentlichen funktionsgleiche Elemente sind mit den gleichen Bezugszeichen versehen; zur besseren Identifikation sind die unterschiedlichen Ausgestaltungen der Kontrollfutterschalen und Signaleinheiten mit entsprechenden "'" gekennzeichnet.

Die in Figur 1 gezeigte Fütterungsanordnung 100 umfasst drei Futterlinien 11, die jeweils eine Förderleitung 10, mehrere Futterschalen 8 sowie jeweils eine Kontrollfutterschale 6 (nur für zwei der drei Futterlinien 11 gezeigt) aufweisen. Ferner weisen die drei Futterlinien 11 jeweils einen Förderantrieb 4 (nur für eine der drei Futterlinien 11 gezeigt) auf, über den eine Förderspirale oder Förderschnecke der Förderleitung 10 angetrieben werden kann, um Futter aus einem Futterbehälter 1 über eine Futtermaschine 3 zu den Futterschalen 8 und der Kontrollfutterschale 6 zu fördern. Wie in Figur 2 zu erkennen ist, können die Futterbehälter 1 optional noch mit einem Aufsatz 2 versehen werden.

Die Futterlinien 11 sind über eine Aufhängung 40 an der Stalldecke befestigt, so dass die Futterlinien 11 in ihrer Höhe verstellt werden können. Dies kann zum einen dazu dienen, die Höhe der Futterschalen 8 und Kontrollfutterschalen 6 auf die Größe der Geflügeltiere anzupassen. Die Höhenverstellbarkeit über die Aufhängung 40 dient insbesondere aber dazu, zu Reinigungszwecken die Futterlinien 11 deutlich anheben zu können.

Futter wird in einem Futtersilo 20, meist außerhalb des Stalls 101, bevorratet. Das Futtersilo 20 ist über eine Verteilleitung 50 mit den Futterbehältern 1 verbunden, so dass bei Antrieb einer Förderspirale oder Förderschnecke der Verteilleitung 50 mittels des Verteilantriebs 30 Futter vom Futtersilo 20 in die Futterbehälter 1 der Futterlinien 11 gefördert werden kann.

Über den in den Figuren 2 und 4 zu erkennenden Füllstandssensor 5 kann in den Kontrollfutterschalen 6 der Füllstand in der Kontrollfutterschale 6 überwacht werden. In Abhängigkeit von diesem Füllstand kann der Füllstandssensor 5 ein Antriebssteuerungssignal generieren, über das die Förderantriebe der Futterlinien 11 gesteuert werden. Sinkt der Füllstand in einer Kontrollfutterschale 6 unter einen vorbestimmten Mindestfüllstandswert, so kann ein Antriebssteuersignal generiert werden, welches die Förderantriebe 4 aktiviert, so dass Futter durch die Förderleitungen 10 zu den Futterschalen 8 und den Kontrollfutterschalen 6 gefördert wird. Zeigt der Füllstandssensor 5 einer Kontrollfutterschale 6 an, dass ein maximaler Futterstand erreicht wurde, kann ein weiteres Antriebssteuersignal generiert werden, mit dem die Förderantriebe 4 und damit die Zufuhr von Futter zu den Futterschalen 8 und Kontrollfutterschalen 6 über die Förderleitungen 10 gestoppt wird. Die vom Füllstandssensor generierten Antriebssteuerungssignale dienen somit vorzugsweise dazu, die Förderantriebe 4 anzusteuern. Alternativ oder zusätzlich können die vom Füllstandssensor 5 generierten Antriebssteuersignale auch dazu verwendet werden, den Verteilantrieb 30 zu steuern. Alternativ oder zusätzlich kann der Verteilantrieb 30 auch von Füllstandssensoren (nicht dargestellt) in den Futterbehältern 1 gesteuert werden.

Die Kontrollfutterschalen 6 sind über Montageelemente 61 schwenkbar oder fest an den Förderleitungen 10 montiert. Über Öffnungen (nicht dargestellt) in der Förderleitung 10 gelangt Futter durch den Futterschacht 62 in einen Futterteller 63, der einen Kragen 64 aufweist, um Futterverluste zu verringern oder zu verhindern. Die Kontrollfutterschale 6 weist ferner eine Stützhaube mit Armen 65 auf.

Die folgenden in Figur 3 zu erkennenden Bestandteile der Kontrollfutterschale 6 sind im Wesentlich in der Regel auch Bestandteil einer Futterschale 8: Montageelement 61, Futterschacht 62, Futterteller 63 mit Kragen 64 und Stützhaube mit Armen 65.

Vom Montageelement 61 der Kontrollfutterschale 6 ist, wie in Figur 4 zu erkennen, ein Füllstandssensor 5 abgehängt. Der Füllstandssensor 5 ragt in dem in Figur 3 gezeigten Zustand in den Futterschacht 62 und kann dort den Füllstand im Futterschacht 62 detektieren. Über eine Leitung 9 ist der Füllstandssensor 5 mit dem Förderantrieb 4 verbunden, so dass ein vom Füllstandssensor 5 generiertes Antriebssteuersignal über die Leitung 9 zur Ansteuerung des Förderantriebs 4 übermittelt werden kann.

An dem Montagelement 61 ist, wie in den Figuren 2 bis 4 schematisch dargestellt, eine Signaleinheit 7 angeordnet. In den Figuren 2 bis 4 ist die Signaleinheit 7 am Montagelement 61 der Kontrollfutterschale 6 angeordnet. Die Signaleinheit 7 kann aber auch an anderen Teilen der Kontrollfutterschale 6 angeordnet oder als separate Baueinheit ausgebildet und beispielsweise neben den übrigen Elementen der Kontrollfutterschale an der Förderleitung angebracht oder am Stallboden, an der Stallwand oder Stalldecke angeordnet sein und das Futtersignal vorzugsweise derart abgeben, dass es im Bereich der Kontrollfutterschale von den Geflügeltieren wahrnehmbar ist.

In dem in Figur 5 dargestellten Beispiel einer Kontrollfutterschale 6' weist die Signaleinheit 7' mehrere Lichtquellen 7'a auf, die als jeweils als stabförmige LED ausgebildet sind, die am Futterschacht 62 angeordnet sind.

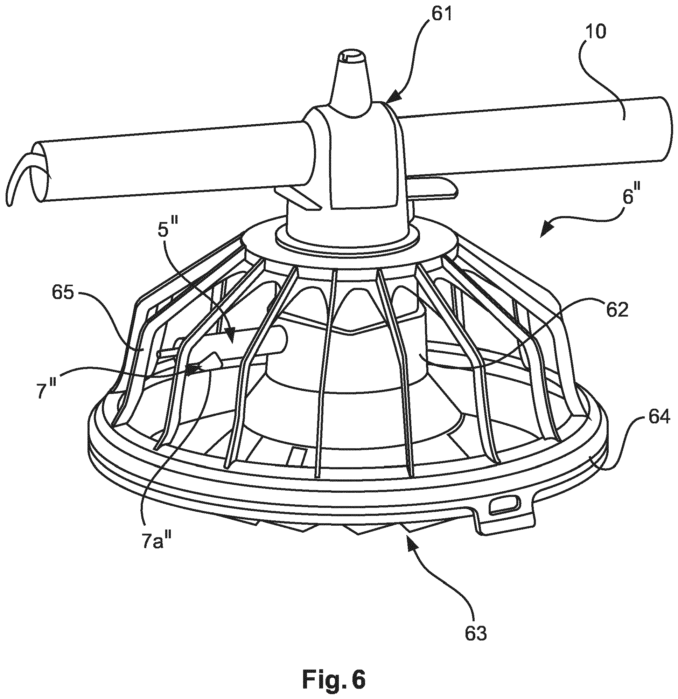

In der in Figur 6 gezeigten Kontrollfutterschale 6" sind ist die Lichtquelle 7"a der Signaleinheit 7"außen an einem in den Futterschacht 62 eingesteckten Füllstandssensor 5" angeordnet. Die Anordnung der Signaleinheit 7" am Füllstandssensor 5" hat unter anderem den Vorteil, dass eine gemeinsame Kabelverbindung leicht zu realisieren ist.

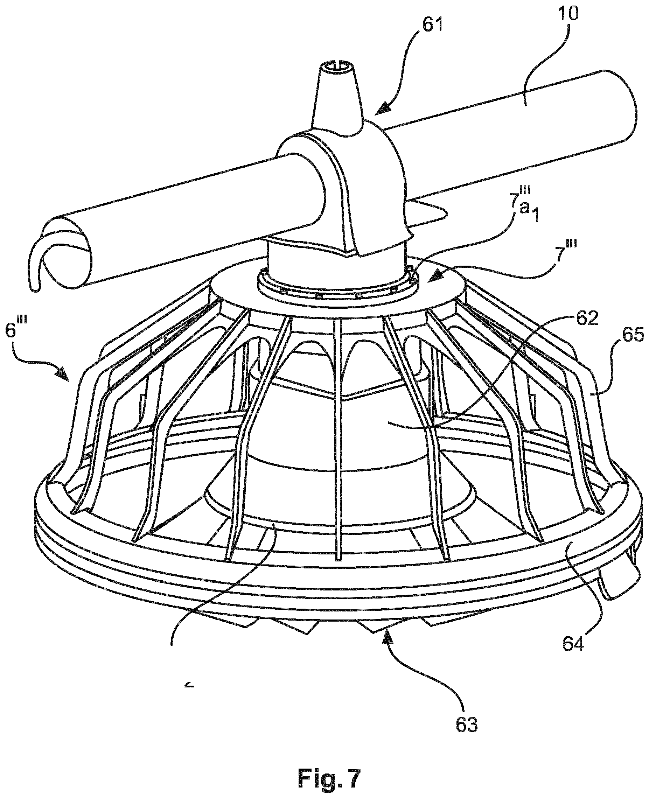

In Figur 7 ist sind mehrere LED 7'''a1 als Lichtquellen der Signaleinheit 7''' an einem oberen Rand des Futterschachts 62 angeordnet, wobei der Futterschacht 62 ganz oder teilweise lichtdurchlässig ausgebildet ist. In dem in Fig. 7 gezeigten Beispiel leuchten die LED 7'''a1 von oben in die lichtdurchlässige Wand des Futterschachts 62 hinein, so dass die Wand des Futterschachts 62 beleuchtet wird. Das Licht kann beispielsweise am unteren Ende bzw. an einer oder mehreren Kanten der Wand des Futterschachts 62 gebrochen werden. Alternativ können eine oder mehrere Lichtquellen auch im Inneren des Futterschachts 62 angeordnet sein, so dass das von den Lichtquellen erzeugte Licht nach außen dringen kann.

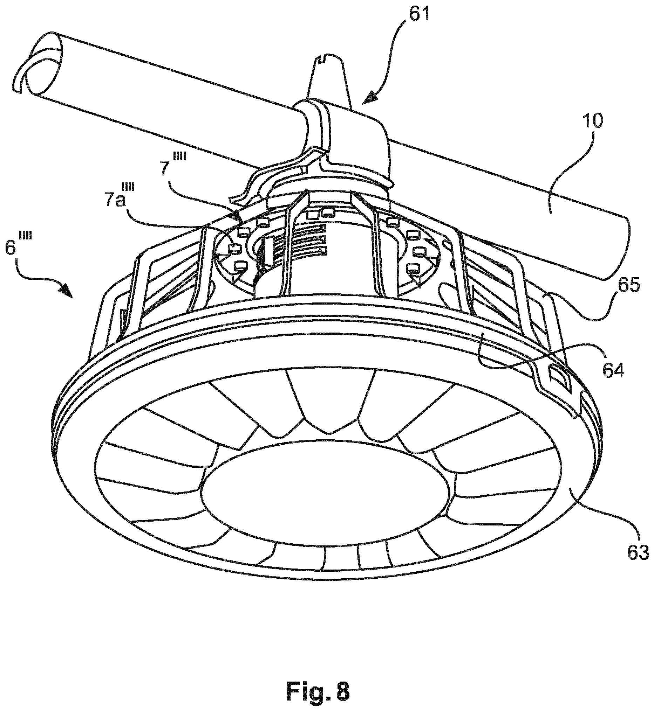

In Figur 8 ist eine Futterschale 6"" dargestellt, bei der die Signaleinheit 7'''' mehrere als LED ausgebildete Lichtquellen 7''''a aufweist, die an einem oberen Verbindungsring der Haltearme 65 angeordnet sind und von dort aus in Richtung des Futtertellers 63 leuchten.

Die Signaleinheit 7, 7', 7", 7''', 7'''' und/oder der Füllstandssensor 5, 5" können, kabelgebunden oder kabellos, mit einer zentralen Steuerungseinheit (nicht dargestellt) der Fütterungsanordnung 100 verbunden sein.

Die Signaleinheit 7, 7', 7", 7''', 7'''' umfasst im in den Figuren 2-8 gezeigten Beispielen eine Lichtquelle 7a, 7'a, 7"a und in den Figuren 2-4 zusätzlich einen Lautsprecher 7b. Die Signaleinheit 7, 7', 7", 7''' kann über die Lichtquellen optische Futtersignale und (sofern vorhanden) über eine akustische Signalquelle akustische Futtersignale ausgeben. Als Schallquelle können zusätzlich oder anstelle eines Lautsprechers auch andere akustische Signalquelle zum Einsatz kommen. Vorzugsweise werden die Futtersignale intermittierend ausgegeben, wobei die maximale Dauer zwischen den Futtersignalausgaben in Abhängigkeit eines Steuerungsparameters ermittelt wird. Insbesondere ist die maximale Dauer zwischen den Futtersignalausgaben abhängig von den seit einer letztmaligen Aktivierung der Förderantriebe 4 verstrichenen Zeit.

Als Futtersignale kommen vorzugsweise optische Signale mit Lichtstrahlung, insbesondere blauem und/oder violettem Licht, und/oder ultravioletter Strahlung zum Einsatz sowie zusätzlich oder alternativ spezifische Geräusche. Bevorzugt kann beispielsweise der Lockruf einer Henne als akustisches Signal eingesetzt werden und/oder einfach zu erzeugende Geräusche aus mechanischen Schallquellen.

Durch diese Futtersignale können die Geflügeltiere animiert werden, die Kontrollfutterschalen 6 häufiger aufzusuchen, da durch die Futtersignale die Attraktivität dieser Kontrollfutterschalen 6 gegenüber den übrigen Futterschalen 8 gesteigert werden kann. Damit kann verhindert werden, dass wenig frequentierte Kontrollfutterschalen mit einem hohem Füllstand dazu führen, dass eine Aktivierung der Förderantriebe 4 für einen längeren Zeitraum unterbleibt und es zu einer Unterversorgung der Futterschalen 8 im Stall 101 kommen kann.

## Patentansprüche

1. Fütterungsanordnung (100) für die Geflügelhaltung, umfassend
- Eine Futterlinie (11) mit einer Förderleitung (10), einem Förderantrieb (4), einem Futterbehälter (1), einer Futterschale (8) und einer Kontrollfutterschale (6) umfassend
- ein Montageelement (61) zur Montage der Kontrollfutterschale an einer Förderleitung (10);
- einen Futterteller (63);
- einen Futterschacht (62), über den Futter aus einer Förderleitung in den Futterteller gelangen kann;
- einen Füllstandssensor (5), der angeordnet und ausgebildet ist, ein Antriebsteuersignal zu generieren;
**dadurch gekennzeichnet, dass** die Kontrollfutterschale eine Signaleinheit (7), die angeordnet und ausgebildet ist, ein von Geflügeltieren wahrnehmbares Futtersignal abzugeben, umfasst, um die Attraktivität der Kontrollfutterschale (6) gegenüber der Futterschale (8) zu steigern, sodass die Kontrollfutterschale (6) stärker frequentiert wird.

2. Fütterungsanordnung (6) nach dem vorhergehenden Anspruch,
**dadurch gekennzeichnet, dass** die Signaleinheit (7) ausgebildet ist, ein optisches, akustisches und/oder olfaktorisches Futtersignal und/oder ein Vibrations-Futtersignal abzugeben.

3. Fütterungsanordnung (6) nach mindestens einem der vorhergehenden Ansprüche,
**dadurch gekennzeichnet, dass** die Signaleinheit (7) ausgebildet ist, als Futtersignal eine, vorzugsweise elektromagnetische, Strahlung abzugeben, insbesondere Licht- und/oder Wärmestrahlung und/oder ein von Geflügeltieren wahrnehmbares Magnetfeld zu erzeugen.

4. Fütterungsanordnung (6) nach mindestens einem der vorhergehenden Ansprüche,
**dadurch gekennzeichnet, dass** die Signaleinheit (7) ausgebildet ist, als Futtersignal blaues und/oder violettes Licht und/oder ultraviolette Strahlung abzugeben.

5. Fütterungsanordnung (6) nach mindestens einem der vorhergehenden Ansprüche, dass die Signaleinheit eine Strahlungsquelle, insbesondere eine Lichtquelle, und/oder eine Schallquelle, beispielsweise einen Lautsprecher, umfasst.

6. Fütterungsanordnung (6) nach mindestens einem der vorhergehenden Ansprüche,
**dadurch gekennzeichnet, dass** die Signaleinheit (7) ausgebildet ist, zwei oder mehrere unterschiedliche Futtersignale abzugeben.

7. Fütterungsanordnung (6) nach mindestens einem der vorhergehenden Ansprüche,
**dadurch gekennzeichnet, dass** die Signaleinheit (7) ausgebildet ist, ein Futtersignal und/oder unterschiedliche Futtersignale intermittierend und/oder kontinuierlich abzugeben.

8. Fütterungsanordnung (6) nach mindestens einem der vorhergehenden Ansprüche,
**dadurch gekennzeichnet, dass** die Signaleinheit (7) ausgebildet ist, ein Futtersignal und/oder unterschiedliche Futtersignale in unterschiedlicher Intensität bzw. in unterschiedlichen Intensitäten abzugeben.

9. Fütterungsanordnung (6) nach mindestens einem der vorhergehenden Ansprüche,
**dadurch gekennzeichnet, dass** die Signaleinheit (7) ausgebildet ist, die Dauer und/oder Intensität und/oder Art des Futtersignals und/oder die Dauer einer Pause zwischen Futtersignalabgaben in Abhängigkeit eines Steuerparameters festzulegen und/oder zu variieren.

10. Fütterungsanordnung (6) nach Anspruch 9,
**dadurch gekennzeichnet, dass** der Steuerparameter in der Signaleinheit (7) und/oder dem Füllstandssensor (5) abgespeichert ist und/oder von einer externen Datenerfassungs-, Datenverarbeitungs- und/oder Datenspeichereinheit an die Signaleinheit (7) und/oder den Füllstandssensor (5) übermittelt werden kann.

11. Fütterungsanordnung (6) nach Anspruch 9 oder 10,
**dadurch gekennzeichnet, dass** der Steuerparameter einen, zwei oder mehrere Parameter aus der folgenden Gruppe umfasst:
- Tageszeit,
- Helligkeit und/oder Temperatur im Stall und/oder der Umgebung,
- Anzahl der Geflügeltiere
- Alter und/oder Gewicht und/oder Größe der Geflügeltiere,
- Benutzervorgabe,
- Futter- und/oder Wasserverbrauch,
- Zeitintervall seit der letzten Ansteuerung eines Förderantriebs und/oder eines Verteilantriebs.

12. Fütterungsanordnung (6) nach mindestens einem der vorhergehenden Ansprüche,
**dadurch gekennzeichnet, dass** die Signaleinheit (7) an der Fütterungsanordnung (6), vorzugsweise am Füllstandssensor (5), befestigt und/oder als separate Baueinheit ausgebildet und vorzugsweise getrennt von den übrigen Elementen der Fütterungsanordnung angeordnet ist.

13. Fütterungsanordnung (6) nach mindestens einem der vorhergehenden Ansprüche,
**dadurch gekennzeichnet, dass** die Signaleinheit (7) und der Follstandssensor (5) als integrierte Baueinheit sind.

14. Verfahren zur Fütterung von Geflügeltieren in der Geflügelhaltung,
umfassend die Schritte:
- Bereitstellen einer Fütterungsanordnung (100) nacheinem der vorgehenden Ansprüche;
- Aktivierung des Förderantriebs (4) zum Fördern von Futter durch die Förderleitung (10) zu der Futterschale (8) und der Kontrollfutterschale (6),
- Stoppen des Förderantriebs in Abhängigkeit eines vom Füllstandssensor (5) der Kontrollfutterschale generierten Antriebsteuersignal,
- Abgeben eines von Geflügeltieren wahrnehmbaren Futtersignals mittels einer Signaleinheit (7), um die Attraktivität der Kontrollfutterschale (6) gegenüber der Futterschale (8) zu steigern, sodass die Kontrollfutterschale (6) stärker frequentiert wird.

## Claims

1. Feeding system (100) for poultry keeping, comprising
- a feed line (11) having a conveyor line (10), a conveyor drive (4), a feed container (1), a feeding pan (8) and a control feeding pan (6) comprising
- a mounting element (61) for attaching the control feeding pan to a conveyor line (10);
- a feeding dish (63);
- a feeding shaft (62) via which the feed can be conveyed from a conveyor line into the feeding dish;
- a filling level sensor (5), which is arranged and formed to generate a drive control signal;
**characterised in that** the control feeding pan comprises a signal unit (7), which is arranged and formed to emit a feeding signal that can be perceived by poultry in order to increase the attractiveness of the control feeding pan (6) compared to the feeding pan (8) so that the control feeding pan (6) is frequented more often.

2. Feeding system (6) as claimed in the preceding claim, **characterised in that** the signal unit (7) is designed to emit an optical, acoustic and/or olfactory feeding signal and/or a vibration feeding signal.

3. Feeding system (6) as claimed in at least one of the preceding claims,
**characterised in that** the signal unit (7) is designed to emit, as a feeding signal, preferably electromagnetic radiation, in particular light and/or heat radiation and/or generate a magnetic field that can be perceived by poultry.

4. Feeding system (6) as claimed in at least one of the preceding claims,
**characterised in that** the signal unit (7) is designed to emit blue and/or violet light and/or ultraviolet radiation as the feeding signal.

5. Feeding system (6) as claimed in at least one of the preceding claims,
**characterised in that** the signal unit comprises a radiation source, in particular a light source, and/or a sound source, for example a speaker.

6. Feeding system (6) as claimed in at least one of the preceding claims,
**characterised in that** the signal unit (7) is designed to emit two or several different feeding signals.

7. Feeding system (6) as claimed in at least one of the preceding claims,
**characterised in that** the signal unit (7) is designed to emit a feeding signal and/or different feeding signals intermittently and/or continuously.

8. Feeding system (6) as claimed in at least one of the preceding claims,
**characterised in that** the signal unit (7) is designed to emit a feeding signal and/or different feeding signals having a different intensity or different intensities.

9. Feeding system (6) as claimed in at least one of the preceding claims,
**characterised in that** the signal unit (7) is designed to determine and/or vary the duration and/or intensity and/or type of the feeding signal and/or the duration of a break between feeding signal emissions depending on a control parameter.

10. Feeding system (6) as claimed in claim 9,
**characterised in that** the control parameter is stored in the signal unit (7) and/or the filling level sensor (5) and/or can be transmitted from an external data acquisition unit, data processing unit and/or data storage unit to the signal unit (7) and/or filling level sensor (5).

11. Feeding system (6) as claimed in claim 9 or 10, **characterised in that** the control parameter comprises one, two or several parameters from the following group:
- time of the day,
- brightness and/or temperature in the barn and/or the surroundings,
- number of poultry,
- age and/or weight and/or size of the poultry,
- user preferences,
- feed and/or water consumption,
- time interval since the last control of a conveyor drive and/or a distributor drive.

12. Feeding system (6) as claimed in at least one of the preceding claims,
**characterised in that** the signal unit (7) is attached to the feeding system (6), preferably to the filling level sensor (5), and/or is designed as a separate unit and preferably located separately from the other elements of the feeding system.

13. Feeding system (6) as claimed in at least one of the preceding claims,
**characterised in that** the signal unit (7) and the filling level sensor (5) are integrated units.

14. Method of feeding poultry in poultry keeping, comprising the steps:
- providing a feeding system (100) as claimed in one of the preceding claims,
- activating the conveyor drive (4) to convey feed via the conveyor line (10) to the feeding pan (8) and the control feeding pan (6),
- stopping the conveyor drive according to a drive control signal generated by the filling level sensor (5) of the control feeding pan,
- emitting a feeding signal that can be perceived by poultry by means of a signal unit (7) in order to increase the attractiveness of the control feeding pan (6) compared to the feeding pan (8) so that the control feeding pan (6) is frequented more often.

## Revendications

1. Ensemble d'alimentation (100) pour l'élevage de volailles, comprenant
- une ligne d'aliments (11) pourvue d'un conduit de transport (10), d'un entraînement de transport (4), d'un récipient d'aliments (1), d'une mangeoire (8) et d'une mangeoire commandée (6) comprenant
- un élément de montage (61) servant à monter la mangeoire commandée au niveau d'un conduit de transport (10) ;
- une assiette d'aliments (63) ;
- une cuve d'aliments (62), par l'intermédiaire de laquelle des aliments peuvent parvenir depuis un conduit de transport dans l'assiette d'aliments ;
- un capteur de niveau de remplissage (5), qui est disposé et réalisé afin de générer un signal de commande d'entraînement ;
**caractérisé en ce que** la mangeoire commandée comprend une unité de signal (7), qui est disposée et réalisée afin d'émettre un signal d'aliments pouvant être perçu par des volailles afin d'augmenter l'attractivité de la mangeoire commandée (6) par rapport à la mangeoire (8) si bien que la mangeoire commandée (6) est plus fréquentée.

2. Ensemble d'alimentation (100) selon la revendication précédente,
**caractérisé en ce que** l'unité de signal (7) est réalisée afin d'émettre un signal d'aliment optique, acoustique et/ou olfactif et/ou un signal d'aliment à vibrations.

3. Ensemble d'alimentation (100) selon au moins l'une quelconque des revendications précédentes, **caractérisé en ce que** l'unité de signal (7) est réalisée afin d'émettre en tant que signal d'aliment un rayonnement de préférence électromagnétique, en particulier un rayonnement de lumière et/ou de chaleur et/ou un champ magnétique pouvant être perçu par des volailles.

4. Ensemble d'alimentation (100) selon au moins l'une quelconque des revendications précédentes, **caractérisé en ce que** l'unité de signal (7) est réalisée afin d'émettre en tant que signal d'aliment une lumière bleue et/ou violette et/ou un rayonnement ultraviolet.

5. Ensemble d'alimentation (100) selon au moins l'une quelconque des revendications précédentes, **caractérisé en ce que** l'unité de signal comprend une source de rayonnement, en particulier une source de lumière et/ou une source acoustique, par exemple un haut-parleur.

6. Ensemble d'alimentation (100) selon au moins l'une quelconque des revendications précédentes, **caractérisé en ce que** l'unité de signal (7) est réalisée afin d'émettre deux ou plusieurs signaux d'aliment différents.

7. Ensemble d'alimentation (100) selon au moins l'une quelconque des revendications précédentes, **caractérisé en ce que** l'unité de signal (7) est réalisée afin d'émettre un signal d'aliment et/ou différents signaux d'aliment par intermittence et/ou en continu.

8. Ensemble d'alimentation (100) selon au moins l'une quelconque des revendications précédentes, **caractérisé en ce que** l'unité de signal (7) est réalisée afin d'émettre un signal d'aliment et/ou différents signaux d'aliment à une intensité différente ou à des intensités différentes.

9. Ensemble d'alimentation (100) selon au moins l'une quelconque des revendications précédentes, **caractérisé en ce que** l'unité de signal (7) est réalisée afin de fixer et/ou de faire varier la durée et/ou l'intensité et/ou le type du signal d'aliment et/ou la durée d'une pause entre des émissions de signaux d'aliment en fonction d'un paramètre de commande.

10. Ensemble d'alimentation (100) selon la revendication 9, **caractérisé en ce que** le paramètre de commande est enregistré dans l'unité de signal (7) et/ou dans le capteur de niveau de remplissage (5) et/ou peut être transmis par une unité de détection de données, de traitement de données et/ou de stockage de données externe à l'unité de signal (7) et/ou au capteur de niveau de remplissage (5).

11. Ensemble d'alimentation (100) selon la revendication 9 ou 10, **caractérisé en ce que** le paramètre de commande comprend un, deux ou plusieurs paramètres issus du groupe qui suit :
- l'heure ;
- la luminosité et/ou la température dans la volaillère et/ou dans l'environnement ;
- le nombre de volailles ;
- l'âge et/ou le poids et/ou la taille des volailles ;
- la spécification d'utilisateur ;
- la consommation d'aliments et/ou d'eau ;
- l'intervalle de temps depuis le dernier pilotage d'un entraînement de transport et/ou d'un entraînement de distribution.

12. Ensemble d'alimentation (100) selon au moins l'une quelconque des revendications précédentes, **caractérisé en ce que** l'unité de signal (7) est fixée au niveau de l'ensemble d'alimentation (100), de préférence au niveau du capteur de niveau de remplissage (5) et/ou est réalisée sous la forme d'un module séparé et est disposée de préférence de manière séparée des autres éléments de l'ensemble d'alimentation.

13. Ensemble d'alimentation (100) selon au moins l'une quelconque des revendications précédentes, **caractérisé en ce que** l'unité de signal (7) et le capteur de niveau de remplissage (5) se présentent sous la forme de modules intégrés.

14. Procédé servant à alimenter des volailles pour l'élevage de volailles, comprenant les étapes consistant à :
- fournir un ensemble d'alimentation (100) selon l'une quelconque des revendications précédentes ;
- activer l'entraînement de transport (4) servant à transporter des aliments par le conduit de transport (10) à la mangeoire (8) et la mangeoire commandée (6) ;
- arrêter l'entraînement de transport en fonction d'un signal de commande d'entraînement généré par le capteur de niveau de remplissage (5) de la mangeoire commandée ;
- émettre un signal d'aliment pouvant être perçu par des volailles au moyen d'une unité de signal (7) afin d'augmenter l'attractivité de la mangeoire commandée (6) par rapport à la mangeoire (8) si bien que la mangeoire commandée (6) est plus fréquentée.
